# EUROPEAN PATENT APPLICATION

(11) **EP 2 072 044 A1**
(43) Date of publication of application: **24.06.2009**
(21) Application number: 07150179.5
(22) Date of filing: 19.12.2007
(51) Int. Cl.: A61K 9/48, A61K 9/00, A61K 47/48, A61K 47/36, A61K 47/38

(54) **Pharmaceutical dosage form comprising a liquid or flowable core composition**

(71) Applicant: Abbott GmbH & Co. KG, 65205 Wiesbaden (DE)
(72) Inventor: Rosenberg, Jörg, 67158, Ellerstadt (DE); Mägerlein, Markus, 68199, Mannheim (DE); Breitenbach, Jörg, 68199, Mannheim (DE); Jung, Tina, 68219, Mannheim (DE); Wested, Ulrich, 69198, Schriesheim (DE)
(74) Representative: Reitstötter - Kinzebach

(57) **Abstract**

A pharmaceutical dosage form, comprising a) a liquid or flowable core, b) a shell of a polysaccharide or proteinaceous material completely enclosing said core, the core comprising an active ingredient dissolved in a pharmaceutically acceptable compound of the formula (I) 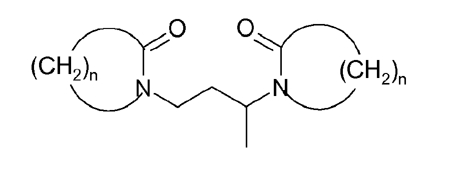 wherein n is an integer from 3 to 5, and wherein the (i) the at least one active ingredient and the compound of the formula (I) account for at least 50 % by weight of the composition; and (ii) the water activity aw of the composition is less than 0.4.

## Description

The present invention relates to a pharmaceutical dosage form comprising a liquid or flowable composition as a core and an enclosing shell.

Liquid oral formulations of active ingredients are often advantageous in that the swift distribution over the gastrointestinal resorption area reduces local concentrations of the active ingredient, thereby facilitating uptake and reducing the danger of irritation.

From a technological point of view, liquid forms are advantageous in that they are naturally more homogenous and can be manufactured without the need for heating or vigorous handling such as kneading, which is particularly beneficial when working with biomolecules such as peptides or proteins which are usually characterized by their low mechanical and thermal stability. Moreover, a significant number of pharmacologically active substances, in particular biomolecules such as peptides or proteins, are either unstable unless in solution or are intrinsically difficult to incorporate into a solid form.

With regard to controlled, sustained and/or targeted release, liquid forms have the additional benefit of allowing the use of liposomes or similar molecular drug delivery systems.

For a variety of reasons, however, such as patient compliance, ease of handling (particularly important in a hospital setting but also when providing treatments directed at children or elderly or disabled persons) and taste-masking, a unit dosage form is usually preferable over a liquid oral solution. Therefore, in many cases pharmaceutically active ingredients in the form of solutions, suspensions or other liquids are dispensed into unit dosage containers such as capsules, a number of which are commercially available.

Today, soft gelatine capsules are mostly manufactured in a stamping process wherein the capsule wall is assembled from two gelatine halves which are stamped out of a gelatine band and then molded. In the widely used Scherer process two endless gelatine bands run against two adjacent and mutually counter-rotating molding rollers. While the gelatine bands are being pressed into the molded and so create the capsule halves, the flowable filler is provided into the thus formed capsule through an exact dosing wedge. Then, capsule halves are sealed together and stamped out.

Recently it was demonstrated that two-part telescoping capsule, usually made of hard gelatine and less frequently of cellulose, provide a reliable alternative to soft gelatine capsules for encapsulating and containing liquid compositions when the seam between the two parts is closed sufficiently tightly, e. g. by welding or sealing (e. g. using the Capsugel^{®} filling and sealing technology or any other suitable methodology described in the art).

Both encapsulation approaches, however, suffer from the fact that many pharmaceutically acceptable solvents, such as the widely used liquid (low molecular weight) polyethylene glycols (e. g. PEG-300 to PEG-400) are largely incompatible with commonly used capsule materials such as gelatine. These capsule materials are generally adjusted to a particular water content and further comprise specific adjuvants, particularly plasticizers, which are likewise indispensable for maintaining the proper mechanical properties of the capsule material, since these mechanical properties must be kept balanced between two extremes: An excess of free water will overly soften and eventually liquefy the capsule material (which is actually the mechanism for release of the active ingredients after administration), whereas exsiccation or extraction of plasticizers will make the capsules brittle. In both cases, the capsules will become prone to breakage even at low mechanical strain, e. g. during deblistering, and thus unusable for practical applications. Thus, solvents which tend to extract water and plasticizers from the capsule material, as do the liquid polyethylene glycols mentioned above, in particular during prolonged periods of storage, have thus to be considered as incompatible with encapsulation of liquids. On the other hand, aqueous solutions will hydratize the capsule material and eventually impair the mechanical integrity of the capsule.

Water migration from the capsule shell to a water-miscible or hygroscopic core composition can largely be avoided by adjusting the initial water concentration in the composition. The water in a liquid core composition for which gelatine capsules do not change their mechanical properties under storage, is called the balanced amount of water (BAW).

Methods for determining the mechanical properties of capsules are known in the art. In particular, texture analysis, which does not entail destruction of the capsules and is therefore especially suitable for in-process sampling, has been described by Kuenz & Röthlisberger, Int. J. Pharm. 236: 145-152, 2002.

However, there are but few alternatives to polyethylene glycols or water as pharmaceutically acceptable solvents. Because of the requirements of pharmaceutical technology, it is not always possible to use a chemically optimal solvent for a given drug.

Problems generally occur when the active ingredient has a limited solubility in the liquid solvent of choice. In particular, during prolonged storage precipitation, e. g. crystallization, may occur, thereby reducing the bioavailability of the drug. Even when the solution remains stable during storage, it is generally desirable to use high concentrations of the active ingredient in order to reduce the volume of the preparation and thus facilitate administration.

There is thus a yet unmet need for a pharmaceutically acceptable solvent which is suitable for a wide range of active ingredients and at the same time compatible with commonly used capsule materials such as gelatine.

WO 2007/050574 describes a composition that comprises a drug of low water solubility in solution in a substantially non-aqueous carrier comprising at least one phospholipid and a pharmaceutically acceptable solubilizing agent. 1,3-Bis-(pyrrolidon-1-yl)-butane (also referred to as vinylpyrrolidone dimer) may be used as solubilising agent in addition to glycols or glyceride materials. The compositions may be encapsulated.

WO 2007/003278 discloses a composition which comprises a solid or semi-solid matrix having at least one active ingredient uniformly dispersed therein, the matrix comprising at least one pharmaceutically acceptable matrix-forming agent and a 1,3-bis-lactamyl-butane.

1,3-bis-lactamyl-butanes have been described as possessing excellent solvent properties for a number of different pharmacologically active ingredients, see WO 98/15291. They are also capable of swelling and solubilizing a number of commonly known polymers such as polyvinyl pyrrolidone (PVP). Quite surprisingly, it has now been found that they are essentially incapable of solubilising polysaccharide or proteinaceous polymers such as gelatine, alginic acid, sodium alginate, etc. Further, it has been found that, although 1,3-bis-(pyrrolidon-1-yl)-butane is miscible with water in any ratio, it does not extract water out of gelatine materials excessively and, hence, does not embrittle the shells of gelatine capsules filled with liquid compositions that contain a large amount of the solvent. Hence, these 1,3-bis-lactamyl-butanes are suitable as essential solvent in the liquid core material of gelatine capsules or capsules made of other polymers of predominantly natural origin. This finding opens intriguing possibilities for the encapsulation of pharmacologically active substances as solutions, using commercially available soft or hard gelatine capsule technology.

The present invention thus relates to a pharmaceutical dosage form, comprising
a) a liquid or flowable composition,
b) a shell of a polysaccharide or proteinaceous material completely enclosing said composition,
the composition comprising an active ingredient dissolved in a pharmaceutically acceptable compound of the formula (I) 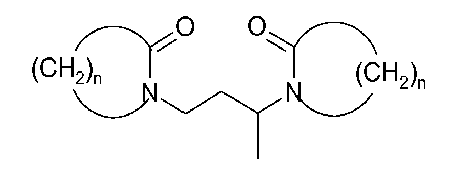 wherein n is an integer from 3 to 5, and wherein the following conditions are satisfied:
(i) the at least one active ingredient and the compound of the formula (I) account for at least 50 % by weight of the composition; and
(ii) the water activity a_{w} of the composition is 0.4 or less.

In certain embodiments of the invention, the water activity a_{w} is 0.3 or less or, particularly, 0.15 or less. The water activity a_{w} may, for example, be as low as 0.01 or 0.05.

Water activity a_{w} is a measure of unbound free water in a system that is available to support chemical (or biological) reactions. It is defined as the vapour pressure of water divided by that of pure water at the same temperature. When a composition is in moisture equilibrium with its environment, its water activity is quantitatively equal to the relative humidity in head space of a container containing the composition, divided by 100. In particular during prolonged periods of storage, an excess of free water will migrate into the capsule shell and soften and eventually liquefy the capsule material. There are several factors that control water activity in a system. Dissolved species, e.g. salts, or sugars, interact with water through dipole-dipole, ionic, and hydrogen bonds. Thus, two compositions with the same water content can vary significantly in their water activities, depending on the amount of free water in the system. Water activity can be measured with commercially available equipment, such as the Pawkit water activity meter available from Decagon Devices, Inc. For the purposes herein, the water activity a_{w} is suitably measured at a temperature of 25 °C.

The water activity a_{w} of the core composition primarily depends on the water content of the composition and on the presence or absence of water activity-depressing solutes in the composition. Generally, dissolved salts will act to depress the water activity a_{w}. Salts may be added to the composition intentionally. Otherwise, the active ingredient may be in the form of an addition salt with a pharmaceutically acceptable acid or base.

According to the invention, the core composition may be adjusted to a particular water content, which is generally up to 12 %, preferably 0.3 to 10 % by weight, relative to the total weight of the composition.

As used herein, the term "pharmaceutical dosage form" is used to denote any formulation in unit dosage form suitable for
- substances exerting pharmacological effects, including medicaments and contraceptives;
- cosmetically advantageous substances; and/or
- nutritional supplements and additives, e. g. vitamins.

As used herein, the term "liquid or flowable" refers to a substance or material which shows such a degree of plastic deformability that the forces caused by the weight of the substance or material alone are sufficient to induce plastic deformation, enabling the application of fluid processing operations such as pouring or stirring, preferably at a temperature not higher than room temperature. Usually the core composition has a viscosity of less than 3000 mPas, preferably less than 1500 mPas at 25 °C.

Pharmaceutically acceptable compounds of formula (I) are known as such. Their preparation is described, inter alia, in WO 98/15291. The following compounds are particularly preferred in the present invention:
1,3-Bis(pyrrolidon-1-yl)butane,
1,3-Bis(piperidon-1-yl)butane,
1,3-Bis(caprolactam-1-yl)butane,
1-(1-Caprolactamyl)-3-(1-piperidonyl)butane,
3-(1-Caprolactamyl)-1-(1-piperidonyl)butane,
1-(1-Caprolactamyl)-3-(1-pyrrolidonyl)butane,
3-(1-Caprolactamyl)-1-(1-pyrrolidonyl)butane,
1-(1-Piperidonyl)-3-(1-pyrrolidonyl)butane,
3-(1-Piperidonyl)-1-(1-pyrrolidonyl)butane,

Most preferably, the compound of formula (I) is 1,3-bis(pyrrolidon-1-yl)-butane.

The compounds of formula (I) are liquid at ambient temperature or liquefy upon gentle heating.

The polysaccharide material may basically be any polysaccharide, in particular a natural polysaccharide or derivative thereof, as long as it is not solubilized by the compound of formula (I) to a significant amount. It is, however, preferred that the polysaccharide is selected from the group consisting of starch, starch derivatives, cellulose, cellulose derivatives, pullulan, sodium alginate, alginic acid, pharmacologically acceptable derivatives thereof and mixtures thereof.

The proteinaceous material preferably is gelatine, i.e. soft or hard gelatine, in particular hard gelatine, e. g. clear and translucent hard gelatine.

Various processes can be used for manufacturing the solid dosage forms according to the invention. These methods usually involve forming a solution of an active ingredient in an appropriate amount of the compound of formula (I) to obtain an active ingredient solution. The active ingredient solution is then either dispensed into a preformed shell or the dispensing of the active ingredient solution and formation of the shell proceed essentially simultaneously.

Procedures for dissolving the active ingredient(s) are well-known to the skilled artisan. Usually, the active ingredient is brought into contact with the compound of formula (I), optionally with agitation or stirring. If desired, the solution may be heated during preparation to accelerate dissolution or to enhance the solubility of the active ingredient. Conveniently, the temperature is in the range of from about 20 °C to about 150 °C, preferably from about 20 °C to about 100 °C; higher temperatures are usually not advisable. In a particularly preferred embodiment of the invention, the preparation procedure is carried out at ambient temperature. Procedures for controlling the content of active ingredient(s) in the resulting solution are likewise well-known to the skilled artisan.

Typically, the active ingredient concentration of the solution is below the value at which the solution is saturated. However, the active ingredient solution may as well be saturated or even supersaturated, i. e., the active ingredient solution may contain suspended or undissolved active ingredient, which may be present in a crystalline or, preferably, essentially amorphous state.

In a preferred embodiment of the invention, the shell is a two-part telescoping capsule, preferably a sealed two-part telescoping capsule. Such capsules as well as the hardware and protocols for manufacturing and handling them have been extensively described in the art, and they are commercially available, e. g. under the brand names of Snap-Fit^{®}, Coni-Snap^{®} and a number of others. In particular, commercially available two-part telescoping capsules, mentioned above, come in a variety of sizes (commonly used codes in order descending size: 000, 00, 0, 1, 2, 3, 4 and 5, among which 0, 1, 2 and 3 are most preferably used) and in a standardized shape which has been optimized for oral administration to human beings.

Preferably, the joint between the cap and the body of the two-part telescoping capsule is subsequently sealed for secure containment of the liquid filler. In a preferred process, a sealing fluid is sprayed onto the joint between the cap and body of the capsule. This lowers the melting point of gelatine in the wetted area. Gentle heat is then applied which fuses the cap to the body of the capsule.

Alternatively, the shell is made of soft gelatine. The formation of soft gelatine capsules is preferably carried out in a stamping process wherein the capsule wall is assembled from two gelatine halves which are stamped out of a gelatine band and then molded. Preferably, the rotary die method such as the Scherer process is used. Herein two endless gelatine bands run against two adjacent and mutually counter-rotating molding rollers. While the gelatine bands are being pressed into the mold and so create the capsule halves, the flowable filler is provided into the thus formed capsule through an exact dosing wedge. There follows the sealing together of the capsule halves, their stamping out, a wash procedure for the freeing of attached oil, a rotational dryer step as well as an adjacent shelf drying.

After filling and closing of the dosage form, the shell may be printed, coloured and/or coated with additional layers to provide a unique and customized appearance. Additional layers dispensed onto the surface of the shell may be of different nature and designed to improve storage stability and aesthetical desirability of the product as well as to the ease of administration.

It is particularly preferred that the core composition is essentially free of solidifying or matrix-forming agents, such as polymers soluble in or swellable by the material of the core as described above. However, in a particular embodiment of the invention the core comprises at least one further ingredient besides the active ingredient or ingredients. Such further ingredients in particular comprise stabilizers, preservatives, tolerability enhancers, resorption enhancers, surfactants, flow regulators, disintegrants, bulking agents, lubricants, effervescent agents, colorants and the like. Such substances are well known to the person skilled in the art.

The compound of formula (I) acts as a non-volatile solvent for the active ingredient and, optionally, further ingredients.

The core composition may contain pharmaceutically acceptable, low molecular weight solvents (having a molecular weight of, e.g., less than 150 Dalton) which are miscible with the compound of formula (I) and which act as a viscosity-depressant. Typically, the composition may contain up to 25 % by weight, for example 5 to 10 % by weight, of the low molecular weight solvent(s). Examples of the pharmaceutically acceptable low molecular weight solvent include alkanols such as ethanol or isopropanol; ketones such as acetone, or lactams such as N-methyl pyrrolidone.

In general, the core comprises 0.001 to 50 % by weight, (preferably 0.1 to 30 % by weight, e.g., 5 to 20 % by weight) of an active ingredient. In general, the compound of formula (I) accounts for at least 45 % by weight of the total weight of the core composition.

As used herein, "active ingredients" are biologically active agents and include those which exert a local physiological effect, as well as those which exert a systemic effect, after oral administration. The invention is particularly useful for water-insoluble or poorly water-soluble (or "lipophilic") compounds. Compounds are considered water-insoluble or poorly water-soluble when their solubility in water (pH 7.0) at 25 °C is less than 10 mg/100 ml, in particular less than 1 mg/100 ml.

Examples of suitable active ingredients include, but are not limited to:
analgesic and anti-inflammatory drugs such as NSAIDs, fentanyl, indomethacin, ibu-profen, ketoprofen, nabumetone, paracetamol, piroxicam, meloxicam, tramadol, and COX-2 inhibitors such as celecoxib and rofecoxib;
anti-arrhythmic drugs such as procainamide, quinidine and verapamil;
antibacterial and antiprotozoal agents such as amoxicillin, ampicillin, benzathine penicillin, benzylpenicillin, cefaclor, cefadroxil, cefprozil, cefuroxime axetil, cephalexin, chloramphenicol, chloroquine, ciprofloxacin, clarithromycin, clavulanic acid, clindamycin, doxyxycline, erythromycin, flucloxacillin sodium, halofantrine, isoniazid, kanamycin sulphate, lincomycin, mefloquine, minocycline, nafcillin sodium, nalidixic acid, neomycin, nortloxacin, ofloxacin, oxacillin, phenoxymethyl-penicillin potassium, pyrimethamine-sulfadoxime and streptomycin;
anti-coagulants such as warfarin;
antidepressants such as amitriptyline, amoxapine, butriptyline, clomipramine, desipramine, dothiepin, doxepin, fluoxetine, reboxetine, amineptine, selegiline, gepirone, imipramine, lithium carbonate, mianserin, milnacipran, nortriptyline, paroxetine, sertraline and 3-[2-[3,4-dihydrobenzofuro[3,2-c]pyridin-2(1 H)-yl]ethyl]-2-methyl-4H-pyrido[1,2-a]pyrimidin-4-one;
anti-diabetic drugs such as glibenclamide and metformin;
anti-epileptic drugs such as carbamazepine, clonazepam, ethosuximide, gabapentin, lamotrigine, levetiracetam, phenobarbitone, phenytoin, primidone, tiagabine, topiramate, valpromide and vigabatrin;
antifungal agents such as amphotericin, clotrimazole, econazole, fluconazole, flucytosine, griseofulvin, itraconazole, ketoconazole, miconazole nitrate, nystatin, terbinafine and voriconazole;
antihistamines such as astemizole, cinnarizine, cyproheptadine, decarboethoxyloratadine, fexofenadine, flunarizine, levocabastine, loratadine, norastemizole, oxatomide, promethazine and terfenadine;
anti-hypertensive drugs such as captopril, enalapril, ketanserin, lisinopril, minoxidil, prazosin, ramipril, reserpine, terazosin and telmisartan;
anti-muscarinic agents such as atropine sulphate and hyoscine;
antineoplastic agents and antimetabolites such as platinum compounds, such as cis-platin and carboplatin; taxanes such as paclitaxel and docetaxel; tecans such as camptothecin, irinotecan and topotecan; vinca alkaloids such as vinblastine, vindecine, vincristine and vinorelbine; nucleoside derivatives and folic acid antagonists such as 5-fluorouracil, capecitabine, gemcitabine, mercaptopurine, thioguanine, cladribine and methotrexate; alkylating agents such as the nitrogen mustards, e.g. cyclophosphamide, chlorambucil, chiormethine, iphosphamide, melphalan, or the nitrosoureas, e.g. carmustine, lomustine, or other alkylating agents, e.g. busulphan, dacarbazine, procarbazine, thiotepa; antibiotics such as daunorubicin, doxorubicin, idarubicin, epirubicin, bleomycin, dactinomycin and mitomycin; HER 2 antibody such as trastuzumab; podophyllotoxin derivatives such as etoposide and teniposide; famesyl transferase inhibitors; anthrachinon derivatives such as mitoxantron; RAF kinase inhibitors such as soratenib;
anti-migraine drugs such as alniditan, naratriptan, sumatriptan and ergotamine;
anti-Parkinsonian drugs such as bromocryptine mesylate, levodopa and selegiline;
antipsychotic, hypnotic and sedating agents such as alprazolam, buspirone, chlordiazepoxide, chlorpromazine, clozapine, diazepam, flupenthixol, fluphenazine, flurazepam, 9-hydroxyrisperidone, lorazepam, mazapertine, olanzapine, oxazepam, pimozide, pipamperone, piracetam, promazine, risperidone, selfotel, seroquel, sertindole, sulpiride, temazepam, thiothixene, triazolam, trifluperidol, ziprasidone and zolpidem;
anti-stroke agents such as lubeluzole, lubeluzole oxide, riluzole, aptiganel, eliprodil and remacemide;
antitussives such as dextromethorphan and laevodropropizine;
antivirals such as acyclovir, ganciclovir, loviride, tivirapine, zidovudine, lamivudine, zidovudine/lamivudine, didanosine, zalcitabine, stavudine, abacavir, lopinavir, amprenavir, nevirapine, efavirenz, delavirdine, indinavir, nelfinavir, ritonavir, saquinavir, adefovir and hydroxyurea;
beta-adrenoceptor blocking agents such as atenolol, carvedilol, metoprolol, nebivolol and propanolol;
cardiac inotropic agents such as amrinone, digitoxin, digoxin and milrinone;
corticosteroids such as beclomethasone dipropionate, betamethasone, budesonide, dexamethasone, hydrocortisone, methylprednisolone, prednisolone, prednisone and triamcinolone;
disinfectants such as chlorhexidine;
diuretics such as acetazolamide, furosemide, hydrochlorothiazide and isosorbide;
enzymes;
essential oils such as anethole, anise oil, caraway, cardamom, cassia oil, cineole, cinnamon oil, clove oil, coriander oil, dementholised mint oil, dill oil, eucalyptus oil, eugenol, ginger, lemon oil, mustard oil, neroli oil, nutmeg oil, orange oil, peppermint, sage, spearmint, terpineol and thyme;
gastro-intestinal agents such as cimetidine, cisapride, clebopride, diphenoxylate, domperidone, famotidine, lansoprazole, loperamide, loperamide oxide, mesalazine, metoclopramide, mosapride, nizatidine, norcisapride, olsalazine, omeprazole, pantoprazole, perprazole, prucalopride, rabeprazole, ranitidine, ridogrel and sulphasalazine;
haemostatics such as aminocaproic acid;
lipid regulating agents such as atorvastatin, fenofibrate, fenofibric acid, lovastatin, pravastatin, probucol and simvastatin;
local anaesthetics such as benzocaine and lignocaine;
opioid analgesics such as buprenorphine, codeine, dextromoramide, dihydrocodeine, hydrocodone, oxycodone and morphine;
parasympathomimetics and anti-dementia drugs such as AIT-082, eptastigmine, galanthamine, metrifonate, milameline, neostigmine, physostigmine, tacrine, donepezil, rivastigmine, sabcomeline, talsaclidine, xanomeline, memantine and lazabemide;
peptides and proteins such as antibodies, becaplermin, cyclosporine, tacrolimus, erythropoietin, immunoglobulins and insuline;
sex hormones such as oestrogens: conjugated oestrogens, ethinyloestradiol, mestranol, oestradiol, oestriol, oestrone; progestogens; chlormadinone acetate, cyproterone acetate, 17-deacetyl norgestimate, desogestrel, dienogest, dydrogesterone, ethynodiol diacetate, gestodene, 3-keto desogestrel, levonorgestrel, lynestrenol, medroxy-progesterone acetate, megestrol, norethindrone, norethindrone acetate, norethisterone, norethisterone acetate, norethynodrel, norgestimate, norgestrel, norgestrienone, progesterone and quingestanol acetate;
stimulating agents such as sildenafil and vardenafil;
vasodilators such as amlodipine, buflomedil, amyl nitrite, diltiazem, dipyridamole, glyceryl trinitrate, isosorbide dinitrate, lidoflazine, molsidomine, nicardipine, nifedipine, oxpentifylline and pentaerythritol tetranitrate;
their N-oxides, their pharmaceutically acceptable acid or base addition salts and their stereochemically isomeric forms.

Pharmaceutically acceptable acid addition salts comprise the acid addition salt forms which can be obtained conveniently by treating the base form of the active ingredient with appropriate organic and anorganic acids.

Active ingredients containing an acidic proton may be converted into their non-toxic metal or amine addition salt forms by treatment with appropriate organic and inorganic bases.

The term addition salt also comprises the hydrates and solvent addition forms which the active ingredients are able to form. Examples of such forms are, for example, hydrates, alcoholates and the like.

The N-oxide forms of the active ingredients comprise those active ingredients in which one or several nitrogen atoms are oxidized to the so-called N-oxide.

The term "stereochemically isomeric forms" defines all possible stereoisomeric forms which the active ingredients may possess. In particular, stereogenic centers may have the R- or S-configuration and active ingredients containing one or more double bonds may have the E- or Z-configuration.

Evidently, two or more active ingredients can be incorporated by blending before dissolution in the core material, by dissolving first one of the ingredients and then adding the other, or by dissolving them independently and mixing the solutions. As mentioned above, suitable procedures are well-known to those skilled in the art.

The core composition may also comprise various other additives, for example pharmaceutically acceptable surfactants, colorants; stabilizers such as antioxidants, light stabilizers, radical scavengers and stabilizers against microbial attack. Suitable additives are well known to the person skilled in the art.

In a preferred embodiment, the composition additionally comprises at least one pharmaceutically acceptable surfactant. The term "pharmaceutically acceptable surfactant" as used herein refers to a pharmaceutically acceptable non-ionic surfactant. The surfactant may effectuate an instantaneous emulsification of the active ingredient released from the dosage form and prevent precipitation of the active ingredient in the aqueous fluids of the gastrointestinal tract.

Preferred surfactants are selected from:
polyoxyethylene alkyl ethers, e.g. polyoxyethylene (3) lauryl ether, polyoxyethylene (5) cetyl ether, polyoxyethylene (2) stearyl ether, polyoxyethylene (5) stearyl ether; polyoxyethylene alkylaryl ethers, e.g. polyoxyethylene (2) nonylphenyl ether, polyoxyethylene (3) nonylphenyl ether, polyoxyethylene (4) nonylphenyl ether or polyoxyethylene (3) octylphenyl ether;
polyethylene glycol fatty acid esters, e.g. PEG-200 monolaurate, PEG-200 dilaurate, PEG-300 dilaurate, PEG-400 dilaurate, PEG-300 distearate or PEG-300 dioleate; alkylene glycol fatty acid mono esters, e.g. propylene glycol monolaurate (Lauroglycol®);
sucrose fatty acid esters, e.g. sucrose monostearate, sucrose distearate, sucrose monolaurate or sucrose dilaurate;
sorbitan fatty acid mono esters such as sorbitan monolaurate (Span® 20), sorbitan monooleate, sorbitan monopalmitate (Span® 40), or sorbitan stearate,
polyoxyethylene castor oil derivates, e.g. polyoxyethyleneglycerol triricinoleate or polyoxyl 35 castor oil (Cremophor® EL; BASF Corp.) or polyoxyethyleneglycerol oxystearate such as polyethylenglycol 40 hydrogenated castor oil (Cremophor® RH 40) or polyethylenglycol 60 hydrogenated castor oil (Cremophor® RH 60); or
block copolymers of ethylene oxide and propylene oxide, also known as polyoxyethylene polyoxypropylene block copolymers or polyoxyethylene polypropyleneglycol such as Poloxamer® 124, Poloxamer® 188, Poloxamer® 237, Poloxamer® 388, or Poloxamer® 407 (BASF Wyandotte Corp.); or
mono fatty acid esters of polyoxyethylene (20) sorbitan, e.g. polyoxyethylene (20) sorbitan monooleate (Tween® 80), polyoxyethylene (20) sorbitan monostearate (Tween® 60), polyoxyethylene (20) sorbitan monopalmitate (Tween® 40), polyoxyethylene (20) sorbitan monolaurate (Tween® 20), or mixtures of one or more thereof.

The following examples will serve to further illustrate the invention without limiting it.

### Example

Five samples of 1,3-bis-(pyrrolidon-1-yl)-butane with different water content (0 %, 5 %, 10 %, 15 %, and 20 %) were prepared. 750.00 mg ± 7.00 mg of each solution was filled in gelatine capsules (telescoping cap and body capsules, size 00).

Twenty-four hours after the capsule preparation, the initial values of water activity and mechanical strength of the capsule shell were measured.

Capsules were stored for three months at 25 °C, 60 % relative humidity and 40 °C, 75 % relative humidity, respectively. Then, water activity and mechanical strength of the capsule shell were measured.

Water activity a_{w} was measured using a Pawkit water activity meter available from Decagon Devices, Inc. Two capsules were opened and their content emptied into a sample beaker. The Pawkit was placed on top of the beaker and the measurement was started. After five minutes the reading was recorded. Determinations were carried out in duplicates.

Mechanical strength of the capsules was determined using a texture analyzer (TA.XT2i; Texture Exponent 32 Software; Stable Micro Systems, Godalming, UK) equipped with a 50 kg load cell. The capsule was placed on the base of the texture analyzer. The compression plate (50 mm in diameter) was lowered with a rate of 2.0 mm/s until the probe reaches the capsule. The actual compression test was carried out at a rate of 0.1 mm/s. The force until rupture of the capsule was recorded. From the stress-strain diagrams thus obtained, the slope between 0.22 mm and 0.58 mm was determined as a measure of the capsule strength. Six capsules were tested and the results were averaged; however outliers were disregarded. The results are summarized in the table below.

| | Initial | | 25 °C, 60 % rh | | 40 °C, 75 % rh | |
|---|---|---|---|---|---|---|
| Sample | a_{w} | Slope ± SD [N/mm] | a_{w} | Slope ± SD [N/mm] | a_{w} | Slope ± SD [N/mm] |
| Empty capsule | | 9.71 ± 0.59 | | | | |
| 0% | 0.12 | 13.23 ± 2.19 | 0.15 | 14.46 ± 1.58 | 0.15 | 14.12 ± 1.69 |
| 5% | 0.25 | 11.30 ± 1.38 | 0.26 | 11.48 ± 1.42 | 0.28 | 9.59 ± 3.80 |
| 10% | 0.38 | 11.28 ± 1.67 | 0.38 | 10.44 ± 2.32^{b} | 0.39 | 9.76 ± 3.54 |
| 15 % | 0.49 | 6.56 ± 0.29 | 0.48 | 5.49 ± 2.77^{b,c} | 0.49 | 1.51 ± 1.25 |
| 20 % | 0.58 | 2.99 ± 0.30^{a} | 0.57 | 3.33 ± 1.03^{b,c} | --^{d} | --^{d} |

a) capsule cap ruptured
b) capsule collapsed and leaked
c) capsule cap ruptured before test started
d) measurement not possible because capsules leaked
   SD = standard deviation

The results show that the shell of capsules filled with a composition having a water activity of more than 0.4 was softened to unacceptable levels. It should be noted that in the capsule filled with pure 1,3-bis-(pyrrolidon-1-yl)-butane, a water activity of 0.12 was determined after 24 h. Thus, water apparently migrated out of the shell into the fill composition.

## Claims

1. A pharmaceutical dosage form, comprising
a) a liquid or flowable composition,
b) a shell of a polysaccharide or proteinaceous material completely enclosing said composition,
the composition comprising at least one dissolved active ingredient and a pharmaceutically acceptable compound of the formula (I) 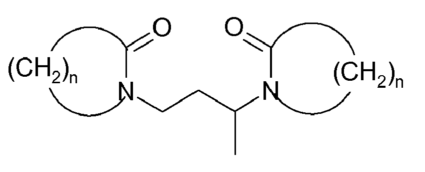 wherein n is an integer from 3 to 5, and wherein the following conditions are satisfied:
(i) the at least one active ingredient and the compound of the formula (I) account for at least 50 % by weight of the composition; and
(ii) the water activity a_{w} of the composition is less than 0.4.

2. The pharmaceutical dosage form of claim 1 wherein the water activity a_{w} of the composition is 0.3 or less.

3. The pharmaceutical dosage form of claim 1 or 2, wherein the water activity a_{w} of the composition is 0.15 or less.

4. The pharmaceutical dosage form of any one of the preceding claims wherein the composition contains up to 12 % by weight of water.

5. The pharmaceutical dosage form of any one of the preceding claims wherein the compound of formula (I) is 1,3-bis-(pyrrolidon-1-yl)-butane.

6. The pharmaceutical dosage form of any one of the preceding claims wherein the polysaccharide material is alginic acid or sodium alginate.

7. The pharmaceutical dosage form of any one of the preceding claims wherein the proteinaceous material is gelatine.

8. The pharmaceutical dosage form of claim 7 wherein the shell is a two-part telescoping capsule.

9. The pharmaceutical dosage form of claim 7, wherein the shell is a soft gelatine capsule prepared by the rotary die method.

10. The pharmaceutical dosage form of any one of the preceding claims wherein the compound of the formula (I) accounts for at least 45 % by weight of the composition.

11. The pharmaceutical formulation of any one of the preceding claims wherein the composition comprises 0.001 to 50 % by weight of active ingredient.

12. The pharmaceutical formulation of any one of the preceding claims wherein the active ingredient has a solubility in water at 25 °C of less than 10 mg/100 ml.

13. The pharmaceutical formulation of any of the preceding claims, wherein the composition additionally comprises at least one pharmaceutically acceptable surfactant.
